# EUROPEAN PATENT APPLICATION

(11) **EP 4 342 495 A1**
(43) Date of publication of application: **27.03.2024**
(21) Application number: 22820347.7
(22) Date of filing: 10.06.2022
(51) Int. Cl.: A61K 47/12, A61K 9/06, A61K 45/00, A61K 47/24

(54) **PREPARATION**

(30) Priority: 11.06.2021 JP 2021098061
(71) Applicant: Kewpie Corporation, Tokyo 150-0002 (JP)
(72) Inventor: AMANO Yohei, Chofu-shi, Tokyo 182-0002 (JP)
(74) Representative: Winter, Brandl - Partnerschaft mbB
(86) International application number: PCT/JP2022/023486
(87) International publication number: WO 2022/260169

(57) **Abstract**

An aspect of the present invention relates to a preparation containing (a) a fatty acid and (b) a phospholipid, in which the mass ratio [(a)/(b)] of the content of the component (a) to the content of the component (b) is 0.5 to 3.0, the total content of the components (a) and (b) is 60 to 100 mass% based on the total amount of the preparation, the content of lipids other than the components (a) and (b) is 30 mass% or less based on the total mass of the preparation, the content of an organic solvent is 30 mass% or less based on the total mass of the preparation, the light transmittance of a water phase at 660 nm when the preparation has been mixed with water 100 times larger than the preparation in volume is 80% or more, and contact with moisture makes it possible to form an oil gel.

## Description

### Technical Field

The present invention relates to a preparation.

### Background Art

A variety of proposals have been made regarding techniques for producing preparations. For example, as a topical bioadhesive preparation, Patent Literature 1 discloses a preparation containing a low-viscosity mixture of a) at least one neutral diacyl lipid containing at least 50% of glycerol dioleate, b) at least one phospholipid composed of at least 50% of phosphatidylcholine and c) 2 to 30 wt% of at least one biocompatible organic solvent containing ethanol, in which the weight ratio between the components a and b is 85:15 to 30:70, contact with an aqueous fluid and/or a body surface makes it possible to form at least one bioadhesive liquid crystal-phase structure, and the viscosity is 0.1 to 5000 mPa·s at 20°C.

### Citation List

### Patent Literature

[Patent Literature 1] Japanese Patent No. 5144277

### Summary of Invention

### Technical Problem

An objective of the present invention is to provide a new preparation capable of forming a robust oil gel by contact with moisture.

### Solution to Problem

The present invention relates to, for example, each of the following inventions.
[1] A preparation containing (a) a fatty acid and (b) a phospholipid,
   in which a mass ratio [(a)/(b)] of a content of the component (a) to a content of the component (b) is 0.5 to 3.0,
   a total content of the components (a) and (b) is 60 to 100 mass% based on a total amount of the preparation,
   a content of lipids other than the components (a) and (b) is 30 mass% or less based on a total mass of the preparation,
   a content of an organic solvent is 30 mass% or less based on the total mass of the preparation,
   a light transmittance of a water phase at 660 nm when the preparation has been mixed with water 100 times larger than the preparation in volume is 80% or more, and
   contact with moisture makes it possible to form an oil gel.
[2] The preparation according to [1], in which the number of carbon atoms in the fatty acid is 8 to 22.
[3] The preparation according to [1] or [2], in which the fatty acid is at least one selected from the group consisting of oleic acid, caprylic acid, capric acid, linoleic acid, docosahexaenoic acid and icosapentaenoic acid.
[4] The preparation according to any one of [1] to [3], in which the phospholipid contains phosphatidylcholine.
[5] The preparation according to [4], in which a content of the phosphatidylcholine is 80 parts by mass or more with respect to 100 parts by mass of the phospholipid.
[6] The preparation according to any one of [1] to [5], in which the mass ratio [(a)/(b)] of the content of the component (a) to the content of the component (b) is 1.0 to 2.5.
[7] The preparation according to any one of [1] to [6], in which a total content of lipids other than the components (a) and (b) is 20 mass% or less based on a total mass of the preparation.
[8] The preparation according to any one of [1] to [7], in which a content of the organic solvent is 15 mass% or less based on a total mass of the preparation.
[9] The preparation according to any one of [1] to [8], in which a light transmittance is 90% or more.
[10] The preparation according to any one of [1] to [9], further containing a physiological active substance.

### Advantageous Effects of Invention

According to the present invention, a new preparation capable of forming a robust oil gel by contact with moisture is provided.

According to a preparation according to the present embodiment, it is possible to form a bioadhesive oil gel. In addition, according to the preparation according to the present embodiment, it is possible to form an oil gel in which an immediate release (burst) of a physiological active substance has been reduced.

### Brief Description of Drawings

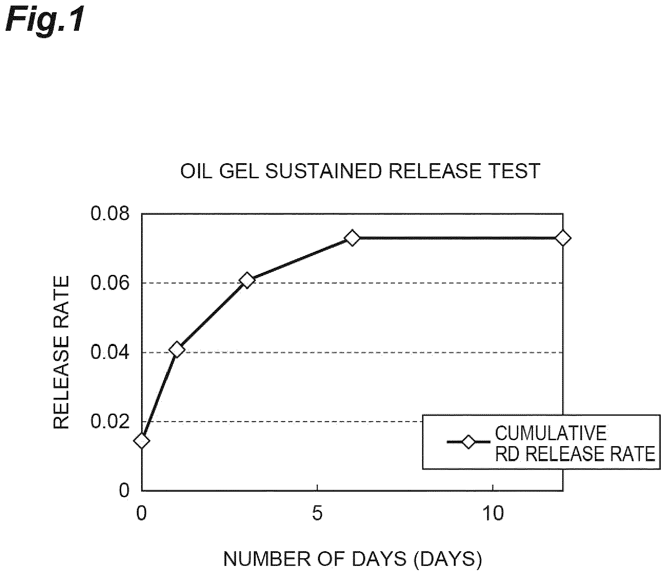

FIG. 1 is a graph showing the result of a sustained release test of an oil gel.

### Description of Embodiments

Hereinafter, an embodiment of the present invention will be described in detail. However, the present invention is not limited to the following embodiment.

### <Features of present invention>

The present invention provides a preparation containing (a) a fatty acid and (b) a phospholipid, in which the mass ratio [(a)/(b)] of the content of the component (a) to the content of the component (b) is 0.5 to 3.0, the total content of the components (a) and (b) is 60 to 100 mass% based on the total amount of the preparation, the content of lipids other than the components (a) and (b) is 30 mass% or less based on the total mass of the preparation, the content of an organic solvent is 30 mass% or less based on the total mass of the preparation, the light transmittance of a water phase at 660 nm when the preparation has been mixed with water 100 times larger than the preparation in volume is 80% or more, and contact with moisture makes it possible to form an oil gel.

### <(a) Fatty acid>

A fatty acid is a chain monocarboxylic acid having one carboxy group (-COOH). The fatty acid may be a saturated fatty acid or may be an unsaturated fatty acid having at least one double bond in a carbon chain. The number of double bonds in the unsaturated fatty acid may be one or more or may be 10 or less or six or less.

### (Number of carbon atoms in fatty acid)

The number of carbon atoms in the fatty acid may be eight or more, 11 or more, 14 or more or 17 or more from the viewpoint of enabling the formation of a more robust oil gel. The number of carbon atoms in the fatty acid may be 8 to 22, 11 to 22, 14 to 22 or 17 to 22 from the viewpoint of enabling the formation of a more robust oil gel.

### (Kind of fatty acid)

Examples of the fatty acid include oleic acid, caprylic acid, capric acid, linoleic acid, docosahexaenoic acid, lauric acid, myristic acid, palmitic acid, stearic acid, arachidic acid, behenic acid, palmitoleic acid, elaidic acid, vaccenic acid, erucic acid, linolenic acid, arachidonic acid and icosapentaenoic acid. The fatty acid is preferably at least one selected from the group consisting of oleic acid, caprylic acid, capric acid, linoleic acid, docosahexaenoic acid and icosapentaenoic acid from the viewpoint of enabling the formation of a more robust oil gel.

### (Content of fatty acid)

The content of the fatty acid may be 15 mass% or more, 20 mass% or more, 25 mass% or more, 30 mass% or more, 35 mass% or more or 40 mass% or more based on the total amount of the preparation. The content of the fatty acid may be 70 mass% or less, 65 mass% or less, 60 mass% or less, 55 mass% or less or 50 mass% or less based on the total amount of the preparation.

### <(b) Phospholipid>

The phospholipid may be, for example, a glycerophospholipid having glycerin as the skeleton or may be a sphingophospholipid having sphingosine as the skeleton. Examples of the glycerophospholipid include phosphatidylcholine, phosphatidylethanolamine, phosphatidylserine, phosphatidylinositol, phosphatidic acid and phosphatidyl glycerol. Examples of the sphingophospholipid include sphingomyelin. One phospholipid may be used singly or two or more phospholipids may be used in combination. The phospholipid preferably contains phosphatidylcholine from the viewpoint of the effect of the present invention being superior.

### (Content of phospholipid)

The content of the phospholipid may be 15 mass% or more, 20 mass% or more, 25 mass% or more, 30 mass% or more, 35 mass% or more or 40 mass% or more based on the total amount of the preparation. The content of the fatty acid may be 70 mass% or less, 65 mass% or less, 60 mass% or less, 55 mass% or less or 50 mass% or less based on the total amount of the preparation.

### (Content of phosphatidylcholine)

The content of phosphatidylcholine may be 70 parts by mass or more, 75 parts by mass or more or 80 parts by mass or more, may be 85 parts by mass or more, 90 parts by mass or more or 95 parts by mass or more and may be 100 parts by mass or less or 99 parts by mass or less with respect to 100 parts by mass of the phospholipid from the viewpoint of enabling the formation of a more robust oil gel.

### (Source of phospholipid)

As the phospholipid, for example, egg yolk lecithin PC-98T (manufactured by Kewpie Corporation) and egg yolk lecithin PL-100M (manufactured by Kewpie Corporation) can be used.

### <Mass ratio of component (a) to component (b)>

The mass ratio [(a)/(b)] of the content (mass%) of the component (a) to the content (mass%) of the component (b) is 0.5 to 3.0 and may be 0.5 to 2.5, 0.75 to 2.5, 1.0 to 2.5, 0.5 to 2.0, 0.75 to 2.0, 1.0 to 2.0, 0.5 to 1.5, 0.75 to 1.5 or 1.0 to 1.5 from the viewpoint of enabling the formation of a more robust oil gel and the viewpoint of enabling the formation of an oil gel in which the immediate release (burst) of a physiological active substance has been further reduced.

### <Total content of components (a) and (b)>

The total content of the components (a) and the component (b) is 60 to 100 mass% based on the total amount of the preparation. The lower limit of the total content of the components (a) and (b) may be 65 mass% or more, 70 mass% or more, 75 mass% or more, 80 mass% or more, 85 mass% or more, 90 mass% or more or 95 mass% or more based on the total amount of the preparation from the viewpoint of enabling the formation of a more robust oil gel. The upper limit of the total content of the components (a) and (b) may be 99 mass% or less, 95 mass% or less, 90 mass% or less, 85 mass% or less, 80 mass% or less, 75 mass% or less or 70 mass% or less based on the total amount of the preparation.

### <Content of lipids other than components (a) and (b)>

The preparation according to the present embodiment may or may not contain one or more lipids other than the components (a) and (b). The content of the lipids other than the components (a) and (b) is 30 mass% or less based on the total mass of the preparation and may be 25 mass% or less and is preferably 20 mass% or less or 15 mass% or less from the viewpoint of enabling the formation of a more robust oil gel. In the case of the lipids other than the components (a) and (b) being contained, the content of the lipids other than the components (a) and (b) may be, for example, 5 mass% or more or 10 mass% or more.

### <Mass ratio of content of lipids other than components (a) and (b)>

The mass ratio (lipids other than components (a) and (b)/fatty acid) of the content of the lipids other than the components (a) and (b) to the content of the fatty acid may be less than 1, 4/5 or less, 3/5 or less or 2/5 or less and may be, for example, 0 or more or 1/5 or more from the viewpoint of enabling the formation of a more robust oil gel.

### <Kind of lipids other than components (a) and (b)>

Examples of the lipids other than the components (a) and (b) include cholesterol, α-tocopherol (for example, dl-α-tocopherol), diacylglycerol, monoacylglycerol and triacylglycerol. Examples of the diacylglycerol include glycerol dioleate.

### <Organic solvent>

Organic solvents are a generic term of organic compounds that dissolve substances that do not dissolve in water and are liquid at a normal temperature and a normal pressure. The organic solvent in the present specification does not include compounds corresponding to lipids (the fatty acid, the phospholipid and the other lipids). Examples of the organic solvent include ethanol, propylene glycol, benzene, acetone, dimethylformamide, dimethyl sulfoxide, N-methyl-2-pyrrolidinone, tetrahydrofuran, chloroform and the like. The organic solvent may be at least one selected from the group consisting of ethanol, propylene glycol, acetone, dimethylformamide, dimethyl sulfoxide, N-methyl-2-pyrrolidinone and tetrahydrofuran.

### (Content of organic solvent)

The content of the organic solvent is 30 mass% or less based on the total mass of the preparation, may be 25 mass% or less or 20 mass% or less and is preferably 15 mass% or less, 12 mass% or less, 10 mass% or less, 8 mass% or less or 6 mass% or less from the viewpoint of enabling the formation of a more robust oil gel, the viewpoint of enabling the formation of an oil gel having a higher bioadhesiveness and viewpoint of enabling the formation of an oil gel in which the immediate release (burst) of a physiological active substance has been further reduced. The content of the organic solvent may be 0 mass% or more based on the total mass of the preparation and may be 2 mass% or 4 mass% or more.

### <Physiological active substance>

The preparation according to the present embodiment may contain a physiological active substance. The physiological active substance in the present specification does not include compounds corresponding to lipids (the fatty acid, the phospholipid and the other lipids). The physiological active substance may be water-soluble or poorly water-soluble, and examples thereof include levodopa, cyclosporine A, docetaxel, paclitaxel, capecitabine, oxaliplatin, geftinat, doxorubicin, irinotecan, gemcitabine, pemetrexed, temozolomide, imatinib, vinorelbine, letrozole, teniposide, etoposide, podophyllotoxin, camptothecin, 10-hydroxycamptothecin, 9-hydroxycamptothecin, 7-ethyl-10-hydroxycamptothecin, topotecan, irinotecan, vinblastine, vincristine, vindesine, vinflunine, vinpocetine, norcantharidin, silybin, propofol, florfenicol, mitiglinide, artemisinin, dihydroartemisinin, sirolimus, ibuprofen, nitrendipine, nicardipine, nimodipine, gliclazide, propulsid, felodipine, glibenclamide, acyclovir, oleanolic acid, breviscapine, ferulic acid, paracetamol, palmitoylrizoxin, penclomedine, tamoxifen, navelbine, valproic acid, tacrolimus, amphotericin B, ketoconazole, domperidone, sulpiride, fenofibrate, bezafibrate, azithromycin, itraconazole, miconazole, brimonidine, latanoprost, silybin, erythromycin, roxithromycin, rifaximin, cisapride, cyclosporine, diclofenac acid, felodipine, ibuprofen, indomethacin, acemetacin, nicardipine, nifedipine, terfenadine, theophylline, ketoprofen, furosemide, spironolactone, dipyridamole, piroxicam, mefenamic acid, trichlorothiazide, pindolol, fat-soluble vitamins (for example, vitamin A, vitamin E), carotenoids (for example, lycopene, chlorophyll, lutein, zeaxanthin, astaxanthin, fucoxanthin), polyphenols (for example, flavonols, flavanones, flavones, isoflavones, phenolcarboxylic acids, anthocyanidins, hydroxycinnamic acid derivatives, ellagic acid and the like), coenzyme Q10 and the like. In addition, the physiological active substance may be an antibody pharmaceutical, and examples thereof include infliximab, adalimumab, golimumab, centolizumab pegol, etanercept, tocilizumab, basiliximab, natalizumab, rituximab and the like.

### (Content of physiological active substance)

The content of the physiological active substance can be set as appropriate depending on the kind, effective amount or the like of the physiological active substance. For example, the content of the physiological active substance may be 0.01 mass% or more, 0.1 mass% or more, 0.5 mass% or more, 1.0 mass% or more, 5.0 mass% or more, 10.0 mass% or more or 15.0 mass% or more and may be 40.0 mass% or less, 35.0 mass% or less, 30.0 mass% or less or 25.0 mass% or less based on the total amount of the preparation.

### <Other materials blended>

Other materials blended other than what has been described above that are blended into the preparation of the present invention can be selected as appropriate depending on the dosage form. Examples of the other materials blended include water, starch, diatomaceous earth, talc and the like. One other material blended may be used singly or two or more other materials blended may be used in combination.

### (Content of water)

The content of water may be an amount at which the gelation of the preparation does not proceed. For example, the content of water is preferably 10% or less and more preferably 5% or less based on the total amount of the preparation.

### <Light transmittance>

In the preparation according to the present invention, the light transmittance of a water phase at 660 nm when the preparation has been mixed with water 100 times larger than the preparation in volume is 80% or more and preferably 85% or more, 90% or more, 95% or more, 99% or more or 100%.

### (Method for measuring light transmittance)

The light transmittance at 660 nm is the rate of the intensity of light having a wavelength of 660 nm that has penetrates a water phase to the intensity of light having a wavelength of 660 nm that has entered the water phase when the preparation has been mixed with water 100 times larger than the preparation in volume. The light transmittance at 660 nm can be measured by a method including mixing of the preparation according to the present embodiment and water 100 times larger than the preparation in volume and the measurement of the light transmittance at 660 nm of a water phase after the mixing with a spectrophotometer (for example, UV-2600, Shimadzu Corporation). A specific measurement method is as described in examples to be described below.

### <Oil gel>

The preparation according to the present embodiment is capable of forming an oil gel by contact with moisture. The preparation according to the present embodiment is capable of forming an oil gel by contact with, for example, a body fluid and water present on a body surface.

### (Confirmation of formation of oil gel)

The formation of an oil gel can be visually confirmed. The formation of an oil gel can be confirmed by, for example, mixing the preparation according to the present embodiment with water 100 times larger than the preparation in volume and observing the appearance after the mixing.

### (Bioadhesiveness)

The oil gel that is formed by contact with moisture may be bioadhesive. The oil gel being bioadhesive is confirmed by evaluating the adhesiveness to a model mucosa. A specific evaluation method is as described in the examples to be described below.

### (Physical protection by oil gel)

According to the preparation according to the present embodiment, it is possible to physically protect a portion on which the oil phase has been formed. That is, according to the present embodiment, it is possible to provide a physical protective layer to a subject portion in a living body.

### (Containing and sustained release of physiological active substance by oil gel)

The oil gel that is formed by the preparation according to the present embodiment enables the containing of the above-described physiological active substance (medication or the like) and the sustainable release of the physiological active substance. The oil gel that is formed by the preparation according to the present embodiment is capable of sustainably releasing the contained physiological active substance over a long period of time (for example, six to 12 days).

### <Shape of preparation>

The shape of the preparation according to the present embodiment may be liquid, powder or the like.

### <Use of preparation>

The preparation according to the present embodiment can be suitably used in uses of pharmaceuticals and medical equipment.

### <Sustained-release preparation>

The preparation according to the present embodiment can be suitably used as a preparation capable of the sustained release of a physiological active substance (sustained-release preparation). According to a sustained-release preparation, it is possible to maintain the blood concentration of a physiological active substance in a suitable range over a long period of time.

### <Administration form>

The preparation according to the present embodiment can be used as, for example, an orally administered agent, a subcutaneous injection, an intramuscular injection and an adhesion prevention material.

### <Orally administered agent>

In the case of being used as an orally administered agent, the preparation according to the present embodiment may be used to relieve pain from stomatitis (for example, oral mucositis). When the preparation according to the present embodiment is administered to the vicinity of a painful area due to stomatitis, in the vicinity of the painful area, the preparation comes into contact with water in saliva to form an oil gel. The oil gel physically protects the vicinity of the painful area and enables the relief of the pain. The orally administered agent may or may not contain a physiological active substance.

### <Subcutaneous injection and intramuscular injection>

The preparation according to the present embodiment is capable of forming an oil gel under the skin by contact with water in a body fluid present under the skin and thus can also be suitably used as a subcutaneous injection or a sustained-release preparation for subcutaneous injection. In the case of being used as an intramuscular injection, the preparation according to the present embodiment can be used, for example, in the same embodiment as the subcutaneous injection.

### <Adhesion prevention material>

In the case of being used as an adhesion prevention material, the preparation according to the present embodiment can be used, for example, in the same or similar embodiment as or to the orally administered agent. The administration site of the adhesion prevention material may be the abdominal cavity. In a case where the preparation according to the present embodiment is used as an adhesion prevention material, in the administration site, the preparation comes into contact with a body fluid to form an oil gel, whereby the oil gel physically protects the administration site and enables adhesion prevention. The adhesion prevention material may or may not contain a physiological active substance.

### <Method for producing preparation>

A method for producing the preparation according to the present embodiment includes mixing of the fatty acid (a), the phospholipid (b) and other components that are blended as necessary. The mixing of each component may be performed under heating. The temperature at the time of mixing each component may be, for example, 50°C to 70°C. In addition, the method for producing the preparation according to the present embodiment include the adjustment of the mass ratio of the content of the component (a) to the content of the component (b), the total content of the components (a) and (b), the content of lipids other than the components (a) and (b) and the content of the organic solvent to the above-described ranges.

### [Examples]

Hereinafter, the present invention will be more specifically described based on test examples. However, the present invention is not limited to the following examples. Hereinafter, unless particularly otherwise described, "%" that indicates the content of a component means "mass%."

### <Abbreviations>

The abbreviations and sources of raw materials used in the following test examples are as described below.
(a) Fatty acids
   OA: Oleic acid (oleic acid > 85%, manufactured by Tokyo Chemical Industry Co., Ltd.)
   C8: Caprylic acid (n-octanoic acid, manufactured by Kanto Chemical Co., Inc.)
   C10: Capric acid (n-deconoic acid, manufactured by FUJIFILM Wako Pure Chemical Corporation)
   LA: Linoleic acid (manufactured by FUJIFILM Wako Pure Chemical Corporation)
   DHA: Docosahexaenoic acid (a fatty acid obtained by purifying "DDoil DHA-46" procured from fish oil manufactured by Nippon Suisan Kaisha, Ltd.)
(b) Phospholipids
   PC: Egg yolk lecithin PC-98T (phosphatidylcholine 98%) (manufactured by Kewpie Corporation)
   PL: Egg yolk lecithin PL-100M (phosphatidylcholine 80%) (manufactured by Kewpie Corporation)
(c) Lipids other than phospholipids and fatty acid
   CH: Cholesterol (manufactured by Tokyo Chemical Industry Co., Ltd.)
   VE: dl-α-tocopherol (manufactured by FUJIFILM Wako Pure Chemical Corporation)
   DAG: Glycerol dioleate (manufactured by FUJIFILM Wako Pure Chemical Corporation)
(d) Organic solvents
   PG: Propylene glycol (Maruishi Pharmaceutical Co., Ltd.)
   DMF: Dimethylformamide (manufactured by Kanto Chemical Co., Inc.)
   DMSO: Dimethyl sulfoxide (manufactured by Kanto Chemical Co., Inc.)
   NMP: n-Methyl-2-pyrrolidinone (manufactured by Kanto Chemical Co., Inc.)
   THF: Tetrahydrofuran (manufactured by Kanto Chemical Co., Inc.)
(e) Model drugs and physiological active substances
   MB: Methylene blue (manufactured by FUJIFILM Wako Pure Chemical Corporation)
   RD: Levodopa (manufactured by Tokyo Chemical Industry Co., Ltd.)
   CsA: Cyclosporine A (manufactured by LC Laboratories)

### <Test Example 1-1: Formation of oil gel and evaluation of transmittance>

The raw materials were heated and dissolved at 60°C according to the following compositions to prepare preparations of examples and comparative examples.

The preparation of each of the examples and the comparative examples was added dropwise to water 100 times larger than the preparation in volume and then mixed by inversion 10 times. The mixture was stirred, and then whether or not an oil gel was formed was visually confirmed. A case where the formation of an oil gel was admitted was determined as "O", and a case where the formation of an oil gel was not admitted was determined as "X." In addition, for the turbidity in a water phase, the transmittance of 660 nm light was measured, and the robustness of the oil gel was confirmed. The transmittance of 660 nm light was measured using a spectrophotometer (UV-2600, Shimadzu Corporation).

Table 1 shows the determination results of oil gel formation and the measurement results of the transmittance of light having a wavelength of 660 nm. "OIL/PC" in Table 1 is OA/PC (mass ratio) in the preparations of Comparative Examples 1A and 1B and the examples and DAG/PC (mass ratio) in the preparations of Comparative Examples 1C and 1D.

**[Table 1]**

| Composition (%) | Comparative Example | Examples | | | | | | | Comparative Example | | |
|---|---|---|---|---|---|---|---|---|---|---|---|
| | 1A | 1A | 1B | 1C | 1D | 1E | 1F | 1G | 1B | 1C | 1D |
| OIL/PC | 1/4 | 1/2 | 1/2 | 1/1 | 1/1 | 2/1 | 2/1 | 2/1 | 4/1 | 1/1 | 2/1 |
| PC | 65.5% | 54.5% | 59.2% | 40.0% | 44.4% | 27.3% | 30.0% | 28.8% | 17.3% | 40.0% | 27.3% |
| OA | 16.4% | 27.2% | 29.6% | 40.9% | 44.4% | 56.8% | 60.0% | 57.5% | 64.5% | - | - |
| DAG | - | - | - | - | - | - | - | - | - | 40.9% | 56.8% |
| CH | - | - | - | - | - | - | - | 4.5% | - | - | - |
| Ethanol | 18.1% | 18.3% | 11.2% | 19.1% | 11.2% | 15.9% | 10.0% | 9.2% | 18.2% | 19.1% | 15.9% |
| Properties | O | O | O | O | O | O | O | O | X | O | O |
| Transmission (660 nm) | 0% | 80% | 95% | 99% | 100% | 98% | 100% | 98% | 96% | 0% | 49% |

It was confirmed that the preparations of the examples formed robust oil gels that did not dissolve or disperse in water. Furthermore, when the content of the organic solvent was 15% or less, the light transmittance became 90% or more, and a more preferable result was obtained.

### <Test Example 1-2: Evaluation of adhesiveness>

0.1 g of the preparation shown in Table 2 was added dropwise to a model mucosa (sufficiently hydrated filter paper), and then the sample (dropwise-added preparation) was uniformly applied to the inside of a 2 cm circle and adapted for two minutes to form an oil gel.

In 900 mL of saline at 37 ± 2°C, a paddle was rotated at 100 rpm at a height of 2.5 cm from the oil gel, the time taken for the oil gel to peel off from the model mucosa was measured over time, and the adhesiveness in a wet environment was evaluated. A small amount (0.04%) of myoglobin (MB) was added to the preparation to improve the visibility.

The evaluation results of the adhesiveness are shown in Tables 2 and 3. A case where 80% or more of the oil gel remained at each elapsed time was evaluated as "O", a case where a part of the oil gel peeled off was evaluated as "Δ", and a case where 80% or more of the oil gel peeled off was evaluated as "X."

**[Table 2]**

| Composition (%) | Examples | | | Comparative Examples | |
|---|---|---|---|---|---|
| | 1D | 1E | 1G | 1C | 1D |
| PC | 44.4% | 27.3% | 28.8% | 40.0% | 27.3% |
| OA | 44.4% | 56.8% | 57.5% | - | - |
| DAG | - | - | - | 40.9% | 56.8% |
| CH | - | - | 4.5% | - | - |
| Ethanol | 11.2% | 15.9% | 9.2% | 19.1% | 15.9% |
| MB | Small amount | Small amount | Small amount | Small amount | Small amount |
| Adhesion | > 60 min | 20 min | > 60 min | 5 min | 30 min |

**[Table 3]**

| Elapsed time (min) | 0 | 5 | 10 | 20 | 30 | 40 | 50 | 60 |
|---|---|---|---|---|---|---|---|---|
| Example 1D | O | O | O | O | O | O | O | O |
| Example 1E | O | Δ | Δ | X | - | - | - | - |
| Example 1G | O | O | O | O | O | O | O | O |
| Comparative Example 1C | O | X | - | - | - | - | - | - |
| Comparative Example 1D | O | Δ | Δ | Δ | X | - | - | - |

The preparations of Example 1D and Example 1G remained on the model mucosa (model skin) for the longest time. It was admitted that preparations having a high transmittance at the time of water dilution and forming a robust oil gel were capable of forming an oil gel having a strong adhesion force under physiological conditions. It was found that, in a case where the content of the organic solvent in the preparation was 15 mass% or less, the adhesion force further increased.

### <Test Example 2: Examination of fatty acid>

The raw materials were heated and dissolved at 60°C according to the following compositions to prepare preparations of examples.

The preparation of each example was added dropwise to water 100 times larger than the preparation in volume and then stirred. After the stirring, whether or not an oil gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation), and the robustness of the oil gel was confirmed.

50% DHA is a fatty acid purified by a method to be described below. Mixed fatty acid preparation was performed on DDoil DHA-46 (manufactured by Nippon Suisan Kaisha, Ltd.) according to "unsaponifiable matter" in Standard Methods for the Analysis of Fats, Oils and Related Materials, and a fatty acid having a DHA content of approximately 50% and EPA of approximately 10% was purified.

**[Table 4]**

| Composition (%) | Examples | | | | | | |
|---|---|---|---|---|---|---|---|
| | 2A | 2B | 2C | 2D | 2E | 2F | 2G |
| Number of carbon atoms | C18 | C8 | C10 | C18 | C8/C18 | C10/C18 | C22 |
| PC | 44.4% | 44.4% | 44.4% | 44.4% | 44.4% | 44.4% | 44.4% |
| OA | 44.4% | - | - | - | 22.2% | 22.2% | - |
| C8 | - | 44.4% | - | - | 22.2% | - | - |
| C10 | - | - | 44.4% | - | - | 22.2% | - |
| LA | - | - | - | 44.4% | - | - | - |
| 50% DHA | - | - | - | - | - | - | 44.4% |
| Ethanol | 11.2% | 11.2% | 11.2% | 11.2% | 11.2% | 11.2% | 11.2% |
| Properties | O | O | O | O | O | O | O |
| Transmission (660 nm) | 100% | 90% | 99% | 100% | 99% | 100% | 99% |

It was confirmed that the preparations of the examples formed a robust oil gel that did not dissolve or disperse in water. In a case where a fatty acid having a large number of carbon atoms was contained, there was a tendency that dispersion of the oil gel became more difficult and the transmittance further increased.

### <Test Example 3: Lipids other than free fatty acid and phospholipid>

The raw materials were heated and dissolved at 60°C according to the following compositions to prepare preparations of examples.

The preparation of each of the examples was added dropwise to water 100 times larger than the preparation in volume and then stirred. After the stirring, whether or not an oil gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation), and the robustness of the oil gel was confirmed.

"FFA" in Table 5 indicates a free fatty acid. "Additive:FFA" indicates the mass ratio between lipids other than the fatty acid and the phospholipid and the free fatty acid (OA).

**[Table 5]**

| Composition (%) | Examples | | | | |
|---|---|---|---|---|---|
| | 3A | 3B | 3C | 3D | 3E |
| Additive :FFA | 1:3 | 1:3 | 1:3 | 3:5 | 1:1 |
| PC | 44.4% | 44.4% | 44.4% | 44.4% | 44.4% |
| OA | 33.3% | 33.3% | 33.3% | 27.8% | 22.2% |
| CH | 11.2% | - | - | - | |
| VE | - | 11.2% | - | - | - |
| DAG | - | - | 11.2% | 16.7% | 22.2% |
| Ethanol | 11.1% | 11.1% | 11.1% | 11.1% | 11.2% |
| Properties | O | O | O | O | O |
| Transmission (660 nm) | 99% | 99% | 99% | 89% | 84% |

It was confirmed that the preparations of the examples formed a robust oil gel that did not dissolve or disperse in water. There was a tendency that, as the mass of the additive (lipids other than the phospholipid and the fatty acid) with respect to the mass of oleic acid became smaller, dispersion of the oil gel became more difficult and the transmittance further increased.

### <Test Example 4: Examination of phospholipid>

The raw materials were heated and dissolved at 60°C according to the following compositions to prepare preparations of examples.

The preparation of each of the examples was added dropwise to water 100 times larger than the preparation in volume and then stirred. After the stirring, whether or not an oil gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation), and the robustness of the gel was confirmed.

"PC content" shown in Table 6 indicates the content (unit: parts by mass) of phosphatidylcholine with respect to 100 parts by mass of the total amount of the phospholipids.

**[Table 6]**

| Composition (%) | Examples | | | | |
|---|---|---|---|---|---|
| | 4A | 4B | 4C | 4D | 4E |
| PC content (parts by mass) | 98 parts by mass | 94 parts by mass | 89 parts by mass | 84 parts by mass | 80 parts by mass |
| PC | 44.4% | 36.0% | 21.6% | 10.8% | - |
| PL | - | 9.0% | 23.4% | 34.2% | 45.0% |
| OA | 44.4% | 45.0% | 45.0% | 45.0% | 45.0% |
| Ethanol | 11.2% | 10.0% | 10.0% | 10.0% | 10.0% |
| Properties | O | O | O | O | O |
| Transmission (660 nm) | 100% | 98% | 95% | 94% | 91% |

It was confirmed that the preparations of the examples formed a robust oil gel that did not dissolve or disperse in water. There was a tendency that, as the amount of phosphatidylcholine in lecithin increased, the oil gel became more robust, and the transmission further increased.

### <Test Example 5: Examination of amount of solvent>

The raw materials were heated and dissolved at 60°C according to the following compositions to prepare preparations of examples.

The preparation of each of the examples was added dropwise to water 100 times larger than the preparation in volume and then stirred. After the stirring, whether or not a gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation), and the robustness of the oil gel was confirmed.

**[Table 7]**

| Composition (%) | Example | | | |
|---|---|---|---|---|
| | 5A | | 5B | |
| Content of solvent | 0% | | 11% | |
| PC | | 33.3% | | 44.4% |
| OA | | 66.7% | | 44.4% |
| Ethanol | | - | | 11.2% |
| Properties | O | | O | |
| Transmission (660 nm) | 100% | | 98% | |

It was confirmed that the preparations of the examples formed a robust oil gel that did not dissolve or disperse in water. It was found that the organic solvent was not an essential component for the preparations.

### <Test Example 6: Examination of kind of solvent >

The raw materials were heated and dissolved at 60°C according to the following compositions to prepare preparations of examples.

The preparation of each of the examples was added dropwise to water 100 times larger than the preparation in volume and then stirred. After the stirring, whether or not a gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation), and the robustness of the oil gel was confirmed.

**[Table 8]**

| Composition (%) | Examples | | | | | |
|---|---|---|---|---|---|---|
| | 6A | 6B | 6C | 6D | 6E | 6F |
| Solvent | PG | Acetone | DMF | DMSO | NMP | THF |
| PC | 45.0% | 45.0% | 45.0% | 45.0% | 45.0% | 45.0% |
| Oleic acid (OA) | 45.0% | 45.0% | 45.0% | 45.0% | 45.0% | 45.0% |
| Ethanol | - | 5.0% | 5.0% | 5.0% | 5.0% | 5.0% |
| PG | 10.0% | - | - | - | - | - |
| Acetone | - | 5.0% | - | - | - | - |
| DMF | - | - | 5.0% | - | - | - |
| DMSO | - | - | - | 5.0% | - | - |
| NMP | - | - | - | - | 5.0% | - |
| THF | - | - | - | - | - | 5.0% |
| Properties | O | O | O | O | O | O |
| Transmission (660 nm) | 100% | 100% | 100% | 100% | 100% | 100% |

It was confirmed that the preparations of the examples formed a robust oil gel that did not dissolve or disperse in water. The transmittance did not change depending on the kind of the organic solvent blended. It was indicated that a robust oil gel could be formed regardless of the kind of the organic solvent blended.

### <Test Example 7: Water-soluble drug>

MB was selected as a model drug of a highly water-soluble physiological active substance. The raw materials were heated and dissolved at 60°C according to the following compositions to prepare preparations containing 0.4 mg of MB per gram.

The preparation of each of the examples and a contrast was added dropwise to water 100 times larger than the preparation in volume and then slowly stirred for one hour. After one hour of the stirring, whether or not an oil gel was generated was visually confirmed. A case where the formation of an oil gel was admitted was determined as "O", and a case where the formation of an oil gel was not admitted was determined as "X." In addition, MB released into a water phase after one hour of the stirring was measured with a spectrophotometer (UV-2600, Shimadzu Corporation) (detection wavelength: 664 nm). The absorbance when MB in the preparation was fully released was regarded as 100% of the burst rate, and the burst rate of the preparation of each of the examples and the contrast was calculated from the obtained absorbance.

**[Table 9]**

| Composition (%) | Example | | Contrast |
|---|---|---|---|
| | 7A | 7B | |
| PC | 45.5% | 25.8% | - |
| OA | 45.5% | 53.8% | 56.8% |
| Ethanol | 9.0% | 20.4% | 43.2% |
| MB | 0.04% | 0.04% | 0.04% |
| Properties | O | O | X |
| Burst | 0.7% | 10.2% | 74.3% |

It was confirmed that the preparations of the examples formed a robust oil gel that did not dissolve or disperse in water. It was possible to confirm that burst rarely occurred from the robust oil gels and the robust oil gels had a high aptitude for the base of long-acting injections. Furthermore, it was found that, when the content of the organic solvent in the preparation was 15 mass% or less, the burst rate further reduced.

### <Test Example 8: Powder dispersion>

RD was selected as a model drug of a highly water-soluble physiological active substance. RD was finely pulverized for 10 minutes with a mortar and then used. The raw materials were heated and dissolved at 60°C according to the following composition to prepare a preparation containing 50 mg of the fine RD powder per gram.

The preparation of the example was added dropwise to water 100 times larger than the preparation in volume and then slowly stirred for one hour. After one hour of the stirring, whether or not an oil gel was generated was visually confirmed. A case where the formation of an oil gel was admitted was determined as "O", and a case where the formation of an oil gel was not admitted was determined as "X." In addition, RD released into a water phase after one hour of the stirring was measured with a spectrophotometer (UV-2600, Shimadzu Corporation) (detection wavelength: 254 nm). The absorbance when RD in the preparation was fully released was regarded as 100% of the burst rate, and the burst rate of the preparation of the example was calculated from the obtained absorbance. Furthermore, the cumulative release rate of RD that was released from the preparation of the example was measured in the same manner over 12 days (n = 2). FIG. 1 is a graph showing the cumulative release rate of RD in a sustained release test.

**[Table 10]**

| Composition (%) | Example | | | |
|---|---|---|---|---|
| | 8A | | | |
| PC | 45.0% | | | |
| OA | 45.0% | | | |
| Ethanol | 5.0% | | | |
| RD | 5.0% | | | |
| Properties | O | | | |
| Burst | 1.4% | | | |
| Cumulative release rate | 1D | 3D | 6D | 12D |
| | 4.1% | 6.1% | 7.3% | 7.3% |

It was confirmed that the preparation of the example formed a robust oil gel that did not dissolve or disperse in water. It was possible to confirm that burst rarely occurred from the robust oil gel and even the preparation in which the drug was pulverized and dispersed had a high aptitude for the base of long-acting injections. Furthermore, stops of an increase in the release rate of RD were admitted over six days to 12 days. This indicates the high stability of the oil gel in water, and it is considered that the collapse of the oil gel of the preparation of the example did not cause the elution of the drug. From this result, a possibility that a high concentration of a drug can be contained with a smaller amount of an oil gel for a long period of time was suggested.

### <Test Example 9: Poorly water-soluble drug>

CsA was selected as a model drug of a poorly water-soluble physiological active substance. The raw materials were heated and dissolved at 60°C according to the following composition to prepare a preparation containing 200 mg of CsA per gram.

The preparation of the example was added dropwise to water 100 times larger than the preparation in volume and then slowly stirred for one hour. After one hour of the stirring, whether or not an oil gel was generated was visually confirmed. A case where the formation of an oil gel was admitted was determined as "O", and a case where the formation of an oil gel was not admitted was determined as "X." In addition, for the turbidity in a water phase, the transmittance (660 nm) was measured using a spectrophotometer (UV-2600, Shimadzu Corporation).

**[Table 11]**

| Composition (%) | Example |
|---|---|
| | 9A |
| PC | 33.2% |
| OA | 33.2% |
| Ethanol | 13.6% |
| CsA | 20.0% |
| Properties | O |
| Transmission (660 nm) | 100% |

It was confirmed that the preparation of the example formed a robust oil gel that did not dissolve or disperse in water. Since cyclosporine does not dissolve in water at all, evaluation by the transmission was employed without determining the amount of cyclosporine. As a result, it was suggested that the release of cyclosporine was suppressed.

## Claims

1. A preparation comprising:
(a) a fatty acid, and
(b) a phospholipid,
wherein a mass ratio [(a)/(b)] of a content of the component (a) to a content of the component (b) is 0.5 to 3.0,
a total content of the components (a) and (b) is 60 to 100 mass% based on a total amount of the preparation,
a content of lipids other than the components (a) and (b) is 30 mass% or less based on a total mass of the preparation,
a content of an organic solvent is 30 mass% or less based on the total mass of the preparation,
a light transmittance of a water phase at 660 nm when the preparation has been mixed with water 100 times larger than the preparation in volume is 80% or more, and
contact with moisture makes it possible to form an oil gel.

2. The preparation according to claim 1,
wherein the number of carbon atoms in the fatty acid is 8 to 22.

3. The preparation according to claim 1 or 2,
wherein the fatty acid is at least one selected from the group consisting of oleic acid, caprylic acid, capric acid, linoleic acid, docosahexaenoic acid and icosapentaenoic acid.

4. The preparation according to any one of claims 1 to 3,
wherein the phospholipid comprises phosphatidylcholine.

5. The preparation according to claim 4,
wherein a content of the phosphatidylcholine is 80 parts by mass or more with respect to 100 parts by mass of the phospholipid.

6. The preparation according to any one of claims 1 to 5,
wherein the mass ratio [(a)/(b)] of the content of the component (a) to the content of the component (b) is 1.0 to 2.5.

7. The preparation according to any one of claims 1 to 6,
wherein a total content of lipids other than the components (a) and (b) is 20 mass% or less based on a total mass of the preparation.

8. The preparation according to any one of claims 1 to 7,
wherein a content of the organic solvent is 15 mass% or less based on a total mass of the preparation.

9. The preparation according to any one of claims 1 to 8,
wherein a light transmittance is 90% or more.

10. The preparation according to any one of claims 1 to 9, further comprising:
a physiological active substance.
